# EUROPÄISCHE PATENTSCHRIFT

(11) **EP 0 793 650 B1**
(45) Veröffentlichungstag und Bekanntmachung des Hinweises auf die Patenterteilung: **03.07.2002**
(21) Anmeldenummer: 95937070.1
(22) Anmeldetag: 14.11.1995
(51) Int. Cl.: C07D 201/08

(54) **VERFAHREN ZUR HERSTELLUNG VON CAPROLACTAM**
METHOD OF PRODUCING CAPROLACTAM
PROCEDE DE FABRICATION DE CAPROLACTAME

(30) Priorität: 25.11.1994 DE 4441962
(43) Veröffentlichungstag der Anmeldung: 10.09.1997
(73) Patentinhaber: BASF AKTIENGESELLSCHAFT, 67056 Ludwigshafen (DE)
(72) Erfinder: ACHHAMMER, Günther, D-68309 Mannheim (DE); FUCHS, Eberhard, D-67227 Frankenthal (DE)
(86) Internationale Anmeldenummer: EP9504464
(87) Internationale Veröffentlichungsnummer: WO9616936

(56) Entgegenhaltungen:
- DE-A- 4 319 134
- FR-A- 2 029 540
- GB-A- 1 121 109
- US-A- 2 245 129
- US-A- 2 301 964

## Beschreibung

Die vorliegende Erfindung betrifft ein Verfahren zur Herstellung von Caprolactam durch Umsetzung von 6-Aminocapronsäurenitril mit Wasser bei erhöhter Temperatur.

In der US 4 628 085 wird die Umsetzung von 6-Aminocapronsäurenitril mit Wasser in der Gasphase an einem speziellen sauren Kieselgel (Porasil® A) bei 300°C beschrieben. Durch Verdünnung mit Wasser, Ammoniak und Wasserstoff/Stickstoff kann Caprolactam bei quantitativem Umsatz in einer Selektivität über 95 % erhalten werden. Jedoch tritt bereits innerhalb von 150 h durch Desaktivierung ein merklicher Umsatz- und Selektivitätsrückgang von je mindestens 5 % ein.

Ein ähnliches Gasphasenverfahren wird in der US 4 625 023 beschrieben. Dort wird ein hochverdünnter Gasstrom von 6-Aminocapronsäurenitril, Adipodinitril, Ammoniak, Wasser und Trägergas über ein Kieselgel- und ein Kupfer/Chrom/Barium-Titanoxid-Katalysatorbett geleitet. Die Caprolactamselektivität beträgt 91 % bei 85 % Umsatz.

Beide Verfahren haben den Nachteil, daß der verwendete heterogene Katalysator rasch desaktiviert wird. Weiterhin muß ein hochverdünnter Gasstrom von 6-Aminocapronsäurenitril mit Wasser an diesen heterogenen Katalysatoren zur Reaktion gebracht werden. Damit verbunden sind hohe Energiekosten für die Verdampfung und große Reaktorvolumina.

In der US 2 245 129 wird die Herstellung linearer Polyamide durch Erhitzen einer 50 gew.-%igen wäßrigen Lösung von 6-Aminocapronsäurenitril auf 200°C für 20 h beschrieben. Die Bildung von Caprolactam wird hierbei nicht beobachtet.

Es war deshalb die technische Aufgabe gestellt, ein Verfahren zur Herstellung von Caprolactam auf Basis 6-Aminocapronsäurenitril zur Verfügung zu stellen, das mit hoher Selektivität in Flüssigphase und ohne Katalysator verläuft, insbesondere sollte der Anteil an Nebenprodukten minimiert werden.

Demgemäß wurde ein Verfahren zur Herstellung von Caprolactam durch Umsetzen von 6-Aminocapronsäurenitril mit Wasser bei erhöhter Temperatur gefunden, indem man
(a) eine wäßrige Lösung von 6-Aminocapronsäurenitril in flüssiger Phase ohne Zusatz eines Katalysators in einem Reaktor A erhitzt unter Erhalt einer Mischung I, bestehend aus im wesentlichen Wasser, Caprolactam und einer hochsiedenden Fraktion ("Hochsieder"), dann
(b) das Wasser aus der erhaltenen Mischung I entfernt unter Erhalt einer Mischung II, bestehend im wesentlichen aus Caprolactam und den Hochsiedern, dann
(c) das Caprolactam und die Hochsieder aus Mischung II durch Destillation voneinander trennt, und anschließend entweder
(d1) die Hochsieder analog zu Stufe (a) in einem weiteren Reaktor B erhitzt und anschließend analog zu den Stufen (b) und (c) aufarbeitet unter Erhalt von weiterem Caprolactam, oder
(d2) die Hochsieder unter vermindertem Druck in Gegenwart einer Base in einem Reaktor C erhitzt und den Reaktionsaustrag unter Gewinnung von Caprolactam destillativ aufarbeitet.

Das erfindungsgemäß als Ausgangsmaterial dienende 6-Aminocapronsäurenitril erhält man üblicherweise durch Hydrierung von Adipodinitril nach einem bekannten Verfahren, beispielsweise beschrieben in DE-A 836 938, DE-A 848 654 oder US 5 151 543.

Man kann auch Mischungen in den Reaktor A einführen, die im wesentlichen 6-Aminocapronsäurenitril sowie Hexamethylendiamin, Adipodinitril und/oder Caprolactam enthalten können sowie hochsiedende Fraktionen ("Hochsieder"), die bei der Aufarbeitung des erfindungsgemäß hergestellten Caprolactams anfallen.

Des weiteren setzt man Wasser bevorzugt im Überschuß ein, besonders bevorzugt verwendet man je mol 6-Aminocapronsäurenitril 10 bis 150, insbesondere 20 bis 100 mol Wasser unter Erhalt einer wäßrigen Lösung von 6-Aminocapronsäurenitril. In einer weiteren bevorzugten Ausführungsform verwendet man üblicherweise 5 bis 25 mol Wasser je mol 6-Aminocapronsäurenitril und kann die Lösung im allgemeinen durch Zusatz eines organischen Lösungsmittels auf 5 bis 25 Gew.-% 6-Aminocapronsäurenitril weiter verdünnen.

Als geeignete Lösungsmittel seien beispielsweise genannt:
C₁-C₄-Alkanole wie Methanol, Ethanol, n-, i-Propanol, Butanole, Glykole wie Ethylenglykol, Diethylenglykol, Triethylenglykol, Tetraethylenglykol, Ether wie Methyl-tert.-butylether, Diethylenglykoldiethylether, C₆-C₁₀-Alkane wie n-Hexan, n-Heptan, n-Octan, n-Nonan, n-Decan sowie Cyclohexan, Benzol, Toluol, Xylol, Lactame wie Pyrrolidon, Caprolactam oder N-C₁-C₄-Alkyl-Lactame wie N-Methylpyrrolidon, N-Methylcaprolactam oder N-Ethylcaprolactam.

In einer weiteren Ausführungsform kann man dem Reaktionsgemisch von 0 bis 5, bevorzugt von 0,1 bis 2 Gew.-% Ammoniak, Wasserstoff oder Stickstoff zusetzen.

Erfindungsgemäß führt man die Reaktion in Stufe (a) bei einer Temperatur im Bereich von 200 bis 370, vorzugsweise von 220 bis 350°C, besonders bevorzugt von 240 bis 320°C durch.

Üblicherweise führt man die Reaktion in Stufe (a) unter Druck durch, wobei man den Druck in der Regel im Bereich von 0,1 bis 50, bevorzugt von 5 bis 25 MPa so wählt, daß das Reaktionsgemisch bevorzugt in flüssiger Phase vorliegt.

Die Reaktionsdauer im Reaktor A hängt im wesentlichen von den gewählten Verfahrensparametern ab und liegt beim kontinuierlich durchgeführten Verfahren im allgemeinen im Bereich von 20 bis 180, bevorzugt von 20 bis 90 min. Bei kürzeren Reaktionszeiten sinkt in der Regel der Umsatz, bei längeren Reaktionszeiten bilden sich nach den bisherigen Beobachtungen störende Oligomere.

Die Cyclisierung (Stufe (a)) führt man bevorzugt kontinuierlich in einem Reaktor A, vorzugsweise in einem Rohrreaktor, in Rührkesseln oder Kombinationen davon durch.

Die Cyclisierung (Stufe (a)) kann man auch diskontinuierlich durchführen. Die Reaktionsdauer liegt dann üblicherweise im Bereich von 30 bis 180 min.

Der Austrag aus Reaktor A ist erfindungsgemäß eine Mischung I, bestehend im wesentlichen aus 50 bis 98, vorzugsweise von 80 bis 95 Gew.-% Wasser und von 2 bis 50, vorzugsweise von 5 bis 20 Gew.-% einer Mischung, bestehend im wesentlichen aus von 50 bis 90, vorzugsweise von 65 bis 85 Gew.-% Caprolactam und von 10 bis 50, vorzugsweise von 15 bis 35 Gew.-% einer hochsiedenden Fraktion (im folgenden als "Hochsieder" bezeichnet).

In Stufe (b) entfernt man erfindungsgemäß das in Mischung I enthaltene Wasser nach üblichen Methoden, bevorzugt durch Destillation, unter Erhalt einer Mischung II, bestehend im wesentlichen aus Caprolactam und den Hochsiedern. In einer bevorzugten Ausführungsform führt man eine Destillation unter vermindertem Druck im Bereich von 10 bis 500 mbar, bevorzugt 50 bis 350 mbar aus, wobei die verwendete Vakuumdestillationskolonne von 2 bis 20, bevorzugt von 4 bis 10 theoretische Böden aufweist.

In Stufe (c) trennt man erfindungsgemäß die in Stufe(b) erhaltene Mischung II in eine Caprolactam-haltige Fraktion (Kopfprodukt) und eine die Hochsieder enthaltende Fraktion (Sumpfprodukt) destillativ auf. Bevorzugt arbeitet man bei der Destillation in Stufe (c) bei einem Druck im Bereich von 0,1 bis 100 mbar, bevorzugt von 1 bis 20 mbar. Bezogen auf die eingesetzte Menge an 6-Aminocapronsäurenitril liegt die Ausbeute an Caprolactam nach dieser Destillation nach bisherigen Beobachtungen im Bereich von 50 bis 90, vorzugsweise von 65 bis 85 Gew.-%.

Die in Stufe (c) erhaltenen Hochsieder können erfindungsgemäß auf drei verschiedene Arten aufgearbeitet werden, wobei man entweder
- in Stufe (d1) die Hochsieder analog zu Stufe (a) in einem weiteren Reaktor B erhitzt und anschließend analog zu den Stufen (b) und (c) aufarbeitet unter Erhalt von weiterem Caprolactam, wobei man bevorzugt die Hochsieder mit der 5 bis 25-, vorzugsweise mit der 10 bis 15-fachen Gewichtsmenge an Wasser vermischt und anschließend bei einer Temperatur im Bereich von 250 bis 350, vorzugsweise von 280 bis 320°C im Reaktor B mit einer Verweilzeit im Bereich von 30 bis 120, vorzugsweise von 45 bis 90 min erhitzt, oder
- in Stufe (d2) die Hochsieder unter vermindertem Druck im allgemeinen Bereich von 1 bis 50, vorzugsweise von 1 bis 10 mbar, in Gegenwart einer Base, in der Regel von 1 bis 10, vorzugsweise von 1 bis 3 Gew.-%, in einem Reaktor C, bevorzugt in einem Rohrreaktor, bei einer Temperatur im Bereich von 200 bis 400, vorzugsweise von 280 bis 320°C, erhitzt und den Reaktionsaustrag unter Gewinnung von weiterem Caprolactam, vorzugsweise destillativ, bevorzugt bei einem Druck im Bereich von 1 bis 50, vorzugsweise von 1 bis 10 mbar, aufarbeitet. In einer bevorzugten Ausführungsform verwendet man eine Destillationskolonnemit einer theoretischen Bödenzahl im Bereich von 2 bis 20, besonders bevorzugt von 5 bis 10.

Als Base setzt man bevorzugt Alkalimetallhydroxide wie Natriumhydroxid und Kaliumhydroxid oder eine Mischung davon ein.

Der Vorteil des erfindungsgemäßen Verfahrens liegt darin, daß man Caprolactam mit hoher Selektivität und hoher Ausbeute ausgehend von 6-Aminocapronsäurenitril in Flüssigphase ohne Katalysator mit kurzen Reaktionszeiten erhält.

### Beispiele

### Beispiel 1

Eine Lösung von 10 Gew.-% 6-Aminocapronitril (ACN) in Wasser wurde in einem Rohrreaktor (Volumen 300 ml) auf 300°C erhitzt, wobei die durchschnittliche Verweilzeit 1 h betrug. Im Austrag wurde kein ACN nachgewiesen. Das Produktgemisch (Mischung I) enthielt 90 Gew.-% Wasser und 10 Gew.-% einer Mischung, enthaltend 76 Gew.-% Caprolactam und 24 Gew.-% Hochsieder. Die Mischung I wurde anschließend bei einem Druck im Bereich von 100 bis 300 mbar in einer Vakuumsdestillationskolonne mit 5 theoretischen Böden destilliert, wobei als Kopfprodukt ammoniakhaltiges Wasser und im Kolonnensumpf (Mischung II) Caprolactam und die Hochsieder anfielen. Die Mischung II wurde in einer weiteren Vakuumsdestillationskolonne (Druck im Bereich von 3 bis 10 mbar) in eine Caprolactamfraktion (Kopfprodukt) und eine Hochsiederfraktion (Sumpfprodukt) aufgetrennt. Die so erhaltene Caprolactamausbeute betrugt 74 Gew.-%, da während der Aufarbeitung weitere 2 Gew.-% der oben erhaltenen Caprolactammenge zu Hochsiedern (im wesentlichen oligomere Lactame) umgesetzt wurden.

### Beispiel 2

Die Hochsiederfraktion aus Beispiel 1 wurde mit der 10-fachen Gewichtsmenge Wasser vermischt und in einem separaten Reaktor 1 h bei 300°C erhitzt. Das entstandene Produktgemisch wurde in der gleichen Weise wie in Beispiel 1 beschrieben aufgearbeitet, wobei man 74 Gew.-% Caprolactam erhielt, so daß die Gesamtausbeute an Caprolactam, bezogen auf die eingesetzte Menge an ACN, 93 Gew.-% betrug.

### Beispiel 3

Beispiel 1 wurde wiederholt. Die erhaltenen Hochsieder wurden mit 1 Gew.-% festem Natriumhydroxid versetzt und unter vermindertem Druck bei 5 mbar auf 300°C erhitzt (Reaktor C). Gleichzeitig wurde das dabei gebildete Caprolactam über eine Destillationskolonne mit 5 theoretischen Böden laufend aus dem Gleichgewicht entfernt. Die Ausbeute an Caprolactam betrug 74 %, bezogen auf die Hochsieder, so daß die Gesamtausbeute an Caprolactam, bezogen auf eingesetztes 6-Aminocapronitril 93 % betrug.

## Patentansprüche

1. Verfahren zur Herstellung von Caprolactam durch Umsetzung einer Lösung von 6-Aminocapronsäurenitril mit Wasser in flüssiger Phase bei erhöhter Temperatur, **dadurch gekennzeichnet, daß** man
(a) eine wäßrige Lösung von 6-Aminocapronsäurenitril in flüssiger Phase ohne Zusatz eines Katalysators in einem Reaktor A erhitzt unter Erhalt einer Mischung I, bestehend aus im wesentlichen Wasser, Caprolactam und einer hochsiedenden Fraktion ("Hochsieder"), dann
(b) das Wasser aus der erhaltenen Mischung I entfernt unter Erhalt einer Mischung II, bestehend im wesentlichen aus Caprolactam und den Hochsiedern, dann
(c) das Caprolactam und die Hochsieder aus Mischung II durch Destillation voneinander trennt, und anschließend entweder
(d1) die Hochsieder analog zu Stufe (a) in einem weiteren Reaktor B erhitzt und anschließend analog zu den Stufen (b) und (c) aufarbeitet unter Erhalt von weiterem Caprolactam, oder
(d2) die Hochsieder unter vermindertem Druck in Gegenwart einer Base in einem Reaktor C erhitzt und den Reaktionsaustrag unter Gewinnung von Caprolactam destillativ aufarbeitet.

2. Verfahren nach Anspruch 1, **dadurch gekennzeichnet, daß** man je mol 6-Aminocapronsäurenitril 10 bis 150 mol Wasser einsetzt.

3. Verfahren nach den Ansprüchen 1 oder 2, **dadurch gekennzeichnet, daß** man der Reaktionsmischung in Stufe (a) ein organisches Lösungsmittel zusetzt.

4. Verfahren nach den Ansprüchen 1 bis 3, **dadurch gekennzeichnet, daß** man die Reaktion in Reaktor Abei einer Temperatur im Bereich von 200 bis 370°C durchführt.

5. Verfahren nach den Ansprüchen 1 bis 4, **dadurch gekennzeichnet, daß** man die Reaktion in Reaktor A bei einem Druck im Bereich von 0,1 bis 50 MPa durchführt.

6. Verfahren nach den Ansprüchen 1 bis 5, **dadurch gekennzeichnet, daß** man eine Verweilzeit in Reaktor A im Bereich von 20 bis 180 min einhält.

## Claims

1. A process for the preparation of caprolactam by reacting a solution of 6-aminocapronitrile with water in the liquid phase at elevated temperatures, wherein
(a) an aqueous solution of 6-aminocapronitrile in the liquid phase is heated without the addition of a catalyst in a reactor A to give a mixture I consisting essentially of water, caprolactam and a high-boiling fraction (high boiler), then
(b) the water is removed from the resulting mixture I to give a mixture II consisting essentially of caprolactam and the high boilers, then
(c) the caprolactam and the high boilers from mixture II are separated from one another by distillation, and then either
(d1) the high boilers are heated similarly to stage (a) in a further reactor B and then worked up similarly to stages (b) and (c) to give further caprolactam, or
(d2) the high boilers are heated under reduced pressure in the presence of a base in a reactor C and the reacted mixture is worked up by distillation to give caprolactam.

2. A process as claimed in claim 1, wherein from 10 to 150 mol of water are used per mol of 6-aminocapronitrile.

3. A process as claimed in claim 1 or 2, wherein an organic solvent is added to the reaction mixture in stage (a).

4. A process as claimed in any of claims 1 to 3, wherein the reaction in reactor A is carried out at from 200 to 370°C.

5. A process as claimed in any of claims 1 to 4, wherein the reaction in reactor A is carried out at from 0.1 to 50 MPa.

6. A process as claimed in any of claims 1 to 5, wherein a residence time of from 20 to 180 minutes is maintained in reactor A.

## Revendications

1. Procédé de fabrication de caprolactame par réaction d'une solution de 6-aminocapronitrile avec de l'eau, en phase liquide, à température élevée, **caractérisé en ce que**:
(a) on chauffe une solution aqueuse de 6-aminocapronitrile en phase liquide sans addition de catalyseur, dans un réacteur A, et l'on obtient un mélange I, essentiellement constitué d'eau, de caprolactame et d'une fraction à haut point d'ébullition (fraction "lourde"), puis
(b) on élimine l'eau du mélange I obtenu, avec obtention d'un mélange II essentiellement constitué de caprolactame et de la fraction lourde, puis
(c) on sépare dans le mélange II le caprolactame et la fraction lourde l'un de l'autre, par distillation, après quoi:
(d1) soit on chauffe la fraction lourde comme à l'étape (a) dans un autre réacteur B et l'on poursuit ensuite le traitement comme à l'étape (b) en obtenant encore du caprolactame,
(d2) soit on chauffe la fraction lourde sous pression réduite en présence d'une base dans un réacteur C et on retraite le produit de réaction par distillation en obtenant du caprolactame.

2. Procédé selon la revendication 1, **caractérisé en ce que** l'on met en oeuvre de 10 à 150 moles d'eau par mole de 6-aminocapronitrile.

3. Procédé selon la revendication 1 ou 2, **caractérisé en ce que** l'on ajoute au mélange réactionnel, dans l'étape (a), un solvant organique.

4. Procédé selon les revendications 1 à 3, **caractérisé en ce que** l'on entreprend la réaction dans le réacteur A à une température de l'ordre de 200 à 370°C.

5. Procédé selon les revendications 1 à 4, **caractérisé en ce que** l'on entreprend la réaction dans le réacteur A à une pression de l'ordre de 0,1 à 50 MPa.

6. Procédé selon les revendications 1 à 5, **caractérisé en ce que** l'on maintient un temps de séjour dans le réacteur A de l'ordre de 20 à 180 minutes.
